(19) Europäisches Patentamt
European Patent Office
Office européen des brevets

(11) **EP 1 484 606 A1**

(12) **EUROPÄISCHE PATENTANMELDUNG**

(43) Veröffentlichungstag:
08.12.2004 Patentblatt 2004/50

(51) Int Cl.$^7$: **G01N 27/49**, G01N 27/403

(21) Anmeldenummer: 04101710.4

(22) Anmeldetag: 23.04.2004

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL HR LT LV MK**

(30) Priorität: **21.05.2003 DE 10322894**

(71) Anmelder: **ProMinent Dosiertechnik GmbH**
**69123 Heidelberg (DE)**

(72) Erfinder:
• **Wittkampf, Michael**
  **69221 Dossenheim (DE)**
• **Pinkowski, Alexander**
  **34005 Palencia (ES)**

(74) Vertreter: **Weber, Dieter, Dr. et al**
**Weber, Seiffert, Lieke**
**Postfach 61 45**
**65051 Wiesbaden (DE)**

(54) **Chloritsensor mit Goldelektrode**

(57)    Die vorliegende Erfindung betrifft einen Sensor zur voltammetrischen oder amperometrischen Messung der Chloritkonzentration ($ClO_2^-$) in einer wäßrigen Lösung. Um einen Chloritsensor bereitzustellen, der eine direkte Messung der Chloritkonzentration ohne Probenahme, Abtrennung von Begleitstoffen oder Chemikalienzugabe erlaubt und eine vernachlässigbare Querempfindlichkeit gegen typische Begleitstoffe des Chlorits, wie insbesondere Chlordioxid ($ClO_2$), Chlorat ($ClO_3^-$) und Hypochlorit ($OCl^-$) aufweist, wird erfindungsgemäß vorgeschlagen, daß der Sensor eine Arbeitselektrode aus Gold aufweist.

Fig. 1

**Beschreibung**

**Gegenstand der Erfindung**

Die Erfindung betrifft einen Sensor zur voltammetrischen oder amperometrischen Messung der Chloritkonzentration ($ClO_2^-$) in einer wäßrigen Meßlösung. Spezieller betrifft die Erfindung einen offenen oder membranbedeckten Chloritsensor, der spezifisch die toxischen Chloritionen ($ClO_2^-$) in beispielsweise mit Chlordioxid ($ClO_2$) desinfiziertem Trinkwasser ohne Querempfindlichkeit für übliche Begleitstoffe, wie Chlordioxid ($ClO_2$), Hypochlorit ($OCl^-$) und Chlorat ($ClO_3^-$) quantitativ nachweisen kann und bei hoher Empfindlichkeit eine vernachlässigbare Abhängigkeit vom pH-Wert der Meßlösung im pH-Bereich von 6,0 bis 9,5 aufweist.

**Stand der Technik**

Beim bekannten Chlorit-Säure-Verfahren zur Chlordioxiderzeugung wird durch Umsetzung von Natriumchlorit ($NaClO_2$) mit Säure, meistens Salzsäure, Chlordioxid ($ClO_2$) nach dem folgenden Schema gebildet:

$$5\ ClO_2^- + 4\ H^+ \leftrightarrow 4\ ClO_2 + Cl^- + 2\ H_2O$$

In umgekehrter Reaktion wird bei verschiedenen Prozessen, wie z. B. bei der Desinfektion von Trinkwasser, Chlordioxid ($ClO_2$) eingesetzt. Hierbei entsteht unter anderem Chlorit ($ClO_2^-$), das wie Chlordioxid ebenfalls bakterizid wirkt. Da Chlorit jedoch toxisch ist, lassen verschiedene nationale Richtlinien nur geringe Restkonzentrationen an Chlorit im Trinkwasser zu, wie z. B. 0,2 bis 1 ppm Chlorit. Um diese Grenzwerte einzuhalten, ist es daher notwendig, den Chloritgehalt von Trinkwasser bei der Trinkwasseraufbereitung mit Chlordioxid ständig zu messen.

Eine weitere direkte Anwendung von Chlorit ist der Einsatz als antimikrobieller Prozeßwasserzusatz bei der Verarbeitung von Geflügel, Fleisch oder Meeresfrüchten. Nach der Behandlung mit dem Prozeßwasser werden diese Lebensmittel unter anderem zur Entfernung des Chlorits mit Trinkwasser gespült, um die vorgeschriebenen Chloritgrenzwerte einzuhalten. Auch hier ist eine ständige Messung des Chloritgehalts im Prozeßwasser und/oder Spülwasser erforderlich.

Derzeit stehen für die Chloritbestimmung in einer wäßrigen Meßlösung keine kontinuierlich arbeitenden und zuverlässig messenden Sensoren zur Verfügung. Zur Zeit eingesetzte Meßverfahren sind aufwendig und/oder teuer und arbeiten diskontinuierlich, d.h. sie sind mit einer Probenahme verbunden, wie iodometrische Titration oder der photometrische Nachweis mit DPD-Reagenz, das erfahrungsgemäß bei Anwesenheit von Chlordioxid zu niedrige Chloritwerte liefert. Einige Verfahren erfordern zur Vermeidung einer Störung der Chloritbestimmung durch Begleitstoffe eine der eigentlichen Messung bzw. Bestimmung vorgeschaltete Trennung, wie Ionenchromatographie oder Kapillarelektrophorese.

Eine weitere Anwendung von Chlorit ist die Rauchgaswäsche, bei der Stickoxide mit Hilfe von natriumchlorithaltigen Lösungen aus Rauchgasen entfernt werden. Zur Bestimmung des Chloritgehaltes schlagen das deutsche Gebrauchsmuster DE 85 27 071.7 und das US-Patent 4 767 601 eine Wärmetönungsmessung vor, bei der die Temperaturerhöhung bei einer Reaktion des Chlorits mit einem Hilfsstoff, wie z. B. Schwefeldioxidgas, gemessen wird. Dieses Verfahren ist jedoch unspezifisch und anfällig für Störungen durch Begleitstoffe. Aufgrund des notwendigen Zusatzes eines reagierenden Hilfsstoffes läßt sich das Verfahren zudem auch nicht direkt im Trinkwasserstrom einsetzen, sondern erfordert die Abzweigung eines Teilstromes als Meßflüssigkeit, der dann nach der Messung verworfen werden muß.

Die DE-OS 41 09 909 beschreibt ein Elektrodensystem für die voltammetrische Messung unter Verwendung einer Arbeitselektrode aus Glaskohlenstoff und einer Gegenelektrode aus einem Metall (Platin, Gold, Silber, Titan, Hastelloy C), mit dem es unter anderem möglich sein soll, neben der Chlordioxidkonzentration auch die anfallenden hohen Chloritkonzentrationen in typischen Bleichlösungen der Papier- und Pulpeindustrie bei pH-Werten im Bereich von 2 bis 7 zu bestimmen. In einem Ausführungsbeispiel, bei dem die Spannung als Funktion des pH-Wertes bei konstanter Chloritkonzentration über den pH-Wertbereich von 2 bis 7 gemessen wurde, wurde gezeigt, daß die Spannungsänderung im genannten pH-Wertbereich immerhin 50 mV betrug, was für die angestrebten Messungen der Oxidations- und/oder Reduktionspotentiale immerhin fast eine Größenordnung der Konzentrationsänderung bedeuten würde.

**Aufgabe der Erfindung**

Der vorliegenden Erfindung liegt die Aufgabe zugrunde, einen Chloritsensor bereitzustellen, der eine direkte Messung der Chloritkonzentration ohne Probenahme, Abtrennung von Begleitstoffen oder Chemikalienzugabe erlaubt, eine vernachlässigbare Querempfindlichkeit gegen typische Begleitstoffe des Chlorits, wie insbesondere Chlordioxid ($ClO_2$), Chlorat ($ClO_3^-$) und Hypochlorit ($OCl^-$) aufweist, für einen Nachweis geringer Mengen an Chlorit im Bereich bis 5 ppm geeignet ist und bei hoher Sondensteilheit eine vernachlässigbare Abhängigkeit vom pH-Wert im pH-Wertbereich von 6,0 bis 9,5 besitzt.

**Lösung**

Die erfindungsgemäße Aufgabe wird durch einen Sensor der eingangs genannten Art gelöst, wobei der Sensor eine Arbeitselektrode aus Gold aufweist.

Es wurde überraschend gefunden, daß die Verwendung einer Arbeitselektrode aus Gold bei einem Sensor zur amperometrischen oder voltammetrischen Messung von Chlorit in wässrigen Meßlösungen hohe Polarisationsspannungen ohne Passivierung und ohne Sauerstoffentwicklung an der Arbeitselektrode zuläßt. Unter Passivierung wird hierbei die Bildung von Oberflächenoxiden an der Arbeitselektrode verstanden. Mit dem erfindungsgemäßen Sensor kann ein sehr hohes anodische Potential angewendet werden, welches nur etwa 300 mV unter dem Potential der beginnenden anodischen Passivierung liegt. Überraschend wurde gefunden, daß mit einer solchen amperometrisch oder voltametrisch betriebenen Elektrodenanordnung mit Gold-Arbeitselektrode bei Anwendung derart hoher Potentiale nicht nur die Querempfindlichkeit des gemessenen Signals für typische und teilweise unvermeidliche Begleitstoffe des Chlorits, wie Chlordioxid ($ClO_2$), Chlorat ($ClO_3^-$) und Hypochlorit (HOCl), so weit verringert werden kann, daß sie vernachlässigbar ist. Es wurde auch überraschend gefunden, daß mit dem erfindungsgemäßen Sensor eine äußerst geringe pH-Wertabhängigkeit im pH-Wertbereich von 6,0 bis 9,5 erreicht werden kann.

Eine mögliche Erklärung dafür, daß die Verwendung von Gold als Arbeitselektrode im Gegensatz zu anderen Edelmetallelektroden, wie Platinelektroden, oder Glaskohlenstoffelektroden die vorgenannte geringe Querempfindlichkeit und geringe pH-Wertabhängigkeit liefert, könnte darin zu finden sein, daß Gold bei dem erforderlichen hohen anodischen Potential noch nicht passiviert wird und daran noch keine Sauerstoffentwicklung stattfindet. Unter einem hohen Potential wird ein Potential von etwa 900 bis 1150 mV gegenüber der Normalwasserstoffelektrode (NHE), das per Konvention 0 mV ist, verstanden.

Prinzipiell würde man annehmen, daß auch eine Platin-Arbeitselektrode für den erfindungsgemäßen Zweck geeignet sein sollte. Jedoch hat sich gezeigt, daß die Empfindlichkeit (Steilheit) eines Sensors mit Platinelektrode geringer ist als diejenige eines Sensors mit Gold-Arbeitselektrode, da die Platin-Oberfläche bei den erforderlichen hohen anodischen Potentialen bereits passiviert wird. Weil die für die Sensorfunktion schädliche Sauerstoffentwicklung an oxidbedeckten, passivierten Elektroden stattfindet bzw. andererseits an oxidbedeckten Elektroden typische Elektrodenreaktionen unterdrückt werden, hat die Gold-Arbeitselektrode erhebliche Vorteile gegenüber der Platin-Elektrode.

Noch weniger als eine Platin-Arbeitselektrode eignet sich eine Glaskohlenstoff-Arbeitselektrode für den erfindungsgemäßen Zweck, da diese eine deutliche pH-Wertabhängigkeit des Nullpunkts und somit des gemessenen Signals bei Anwesenheit von Chlorit aufweist.

Der erfindungsgemäße Chloritsensor ist für Chloritionen spezifisch und besitzt kaum Querempfindlichkeit gegen die oben genannten typischen Begleitstoffe des Chlorits. Da der Sensor keine Stoffe in das Meßwasser abgibt, eignet er sich besonders für die Bestimmung des Chloritgehaltes direkt im Trinkwasser, ohne daß die Entnahme einer später zu verwerfenden Probe erforderlich ist. Der erfindungsgemäße Chloritsensor läßt sich kontinuierlich einsetzen, so daß der Chloritgehalt permanent bzw. in kurzen Intervallen und mittels einer entsprechend ausgelegten Erfassungselektronik automatisch gemessen werden kann.

Der erfindungsgemäße Chloritsensor läßt sich voltammetrisch, amperometrisch oder auch cyclovoltammetrisch betreiben. Er kann in jeder üblichen Ausgestaltung bekannter Meßelektrodensysteme bereitgestellt werden, vorzugsweise als Zweielektrodensystem oder Dreielektrodensystem. In einem Dreielektrodensystem umfaßt der Sensor vorteilhafterweise eine Arbeitselektrode aus Gold, eine übliche Referenzelektrode, z. B. eine Silber/Silberchloridelektrode, und eine übliche Gegenelektrode, z. B. eine Platinelektrode. Die Arbeitselektrode aus Gold kann als offene oder membranbedeckte Arbeitselektrode ausgeführt sein. Bei einer "offenen" Ausführungsform der Arbeitselektrode des erfindungsgemäßen Sensors ist die Arbeitselektrode frei zugänglich für den direkten Kontakt mit der Meßlösung ausgebildet.

Zur Messung der Chloritkonzentration mit dem erfindungsgemäßen Chloritsensor in einer wäßrigen Meßlösung wird zwischen der Arbeitselektrode und der Gegenelektrode als Arbeitsspannung zweckmäßigerweise ein konstantes anodisches Potential von +900 bis +1150 mV gegenüber der Normalwasserstoffelektrode angelegt und der bei der Arbeitsspannung fließende Strom gemessen. Bevorzugt liegt die Arbeitsspannung im Bereich von +1000 bis +1100 mV, besonders bevorzugt bei etwa 1000 mV gegenüber der Normalwasserstoffelektrode. Der resultierende Meßstrom wird als amperometrisches, zur Chloritkonzentration proportionales Signal ausgewertet.

Bei einer bevorzugten Ausführungsform des erfindungsgemäßen Sensors ist die Arbeitselektrode aus Gold von der Meßlösung durch eine Membran räumlich getrennt, wobei die Membran vorzugsweise eine hydrophile oder hydrophilisierte Membran ist. Besonders bevorzugt besteht die Membran aus Polyvinylidendifluorid (PVDF) oder Polyethylentherephthalat (PET). Es ist weiterhin zweckmäßig, wenn die Membran eine Porengröße von 0,1 bis 5 μm, vorzugsweise eine Porengröße von 0,2 bis 1,0 μm, besonders bevorzugt eine Porengröße von etwa 0,5 aufweist.

Bei einer besonders bevorzugten Ausführungsform des membranbedeckten erfindungsgemäßen Chloritsensors sind die Elektroden von einer Membrankappe umgeben, welche die Elektroden von der Meßlösung trennt, wobei die Membrankappe mit einem Innenelektrolyten gefüllt ist, der mit den Elektroden in Kontakt ist, und die Membrankappe wenigstens eine Membran aufweist, die den Innenraum der Membrankappe und den Außenraum der Meßlösung trennt. Das flüssigkeitsdichte Material der Membrankappe weist wenigstens eine Öffnung auf, die von der porösen Membran überspannt ist. Der Innenelektrolyt ist mit der Arbeitselektrode und der Membran in Kontakt. Ein Beispiel für ein geeignetes Membranmaterial ist das vorgenannte Polyvinylidendifluorid (PVDF) mit einer Porengröße von etwa 0,5 μm. Andere semipermeable Membranen oder auch Diaphragmen sind erfindungsgemäß ebenfalls geeignet.

Bei der Ausführungsform des erfindungsgemäßen Sensors mit membranbedeckter Arbeitselektrode wird als Innenelektrolyt bevorzugt eine Kaliumchloridlösung (KCl) verwendet. Diese kann vorteilhaft mit einem üblichen Geliermittel, wie z. B. mit Hydroxyethylcellulose, verdickt sein.

Als Arbeitselektrode am erfindungsgemäßen Chloritsensor wird Gold vorzugsweise in Form eines Stiftes mit im wesentlichen kreisförmigem Querschnitt und mit einem Durchmesser von etwa 1 mm bis etwa 5 mm, vorzugsweise etwa 1,5 mm bis 3 mm, besonders bevorzugt etwa 2 mm eingesetzt. Alternativ kann auch ein als Träger dienender, mit Gold plattierter Grundkörper als Elektrode eingesetzt werden, wobei die elektrische Ableitung direkt von der Goldplattierung erfolgt.

Die Arbeitselektrode aus Gold im erfindungsgemäßen Chloritsensor besitzt neben den oben genannten Vorteilen die Eigenschaft, daß sie chemisch und elektrochemisch relativ inert ist und gegenüber anderen Edelmetallelektroden höhere Polarisationsspannungen in wäßrigen Lösungen ohne elektrolytische Wasserzersetzung zuläßt. Die hohen Potentiale, die bei der erfindungsgemäß verwendeten Goldelektrode besonders vorteilhaft im Hinblick auf die Querempfindlichkeit für Begleitstoffe sind, führen bei der Verwendung von anderen bekannten Edelmetallelektroden bereits zum Einsetzen der elektrolytischen Wasserzersetzung, so daß eine Messung mit anderen Edelmetallelektroden bei solch hohen anodischen Potentialen nicht möglich ist.

Der bei der angelegten Arbeitsspannung fließende Strom wird mittels entsprechender Erfassungselektronik, die auf dem Gebiet der Sensorik hinlänglich bekannt und nicht Gegenstand der vorliegenden Erfindung ist, als chloritkonzentrationsproportionales Signal ausgewertet. Der erfindungsgemäße Chloritsensor kann beispielsweise auch cyclovoltammetrisch oder im Potentialsprungverfahren betrieben werden, wobei vorteilhaft ein anodisches Potential im Bereich von -1000 bis +1300 mV gegenüber NHE abgefahren wird.

Bei der vorgenannten Arbeitsspannung der amperometrischen Messung im Bereich von +900 bis +1150 mV gegenüber NHE arbeitet der erfindungsgemäße Sensor im Diffusionsgrenzstrombereich. Dabei läuft folgende Oxidationsreaktion an der Arbeitselektrode ab:

$$ClO_2^- \rightarrow ClO_2 + e^-$$

Diffusionsgrenzstrombereich bedeutet dabei, daß bei dem angelegten Potential der gesamte zur Elektrodenoberfläche diffundierende Analyt umgesetzt wird. Der resultierende Oxidationsstrom kann somit als ein zur Chloritkonzentration proportionales Signal ausgewertet werden.

Neben der direkten amperometrischen Anwendung mit zeitlich konstantem Potential läßt sich der erfindungsgemäße Chloritsensor auch mit dem Verfahren der Cyclovoltammetrie einsetzen. Hierbei wird ein Potentialbereich in Form eines Dreiecks (Hin- und Rückweg) mit einer vorgegebenen Potentialvorschubgeschwindigkeit [mV/s] abgefahren und der dabei fließende Strom gemessen. Die Höhe des Stroms bei einem Potential im diffusionskontrollierten Bereich des Cyclovoltammogrammes ist hierbei wiederum proportional zur Konzentration des Analyten.

Eine weitere Variante ist das Potentialsprungverfahren. Dabei wird ein Potential oder werden mehrere Potentiale oberhalb oder auch unterhalb des eigentlichen Meßpotentials angelegt. Dabei kann mit Vorteil z. B. eine gleichzeitige Regeneration durch Entfernung von Reaktionsprodukten oder adsorbierten Stoffen von der Elektrodenoberfläche erfolgen, indem man in einer bestimmten Abfolge die verschiedenen Potentiale für eine vorgegebene Zeit anlegt und nur beim eigentlichen Meßpotential die Strommessung für die Quantifizierung des Chloritgehalts durchführt.

Die nachfolgende Beschreibung und die dazu gehörigen Figuren erläutern eine besonders bevorzugte Ausführungsform des erfindungsgemäßen Chloritsensors sowie beispielhafte Meßergebnisse mit dem erfindungsgemäßen Sensor und Vergleichsbeispiele.

Figur 1    ist eine schematische Darstellung eines erfindungsgemäßen Chloritsensors als Zweielektrodensystem mit einer membranbedeckten Arbeitselektrode aus Gold.

Figur 2    zeigt Cyclovoltammogramme in Leitungswasser (LW) ohne Chlorit bzw. mit 2,5 ppm Chlorit, jeweils bei einem pH-Wert von 7,2, unter Verwendung des erfindungsgemäßen Chloritsensors gemäß Figur 1.

Figur 3    zeigt die Abhängigkeit des Nullpunktsignals vom pH-Wert von Sensoren mit drei verschiedenen Arbeitselektrodenmaterialien (Gold, Platin und Glaskohlenstoff), die bei gleicher Versuchsanordnung gemessen wurden.

Figur 4    zeigt die Abhängigkeit des Chlorit-Signals vom pH-Wert von Sensoren mit drei verschiedenen Arbeitselektrodenmaterialien (Gold, Platin und Glaskohlenstoff), die bei gleicher Versuchsanordnung gemessen wurden und bei 0,5 ppm Chlorit.

Figur 5     zeigt Kennlinien von Chloritsensoren mit Arbeitselektroden aus Gold bzw. Platin bei konstantem pH-Wert und über einem Chloritbereich von 0 bis 2 ppm.

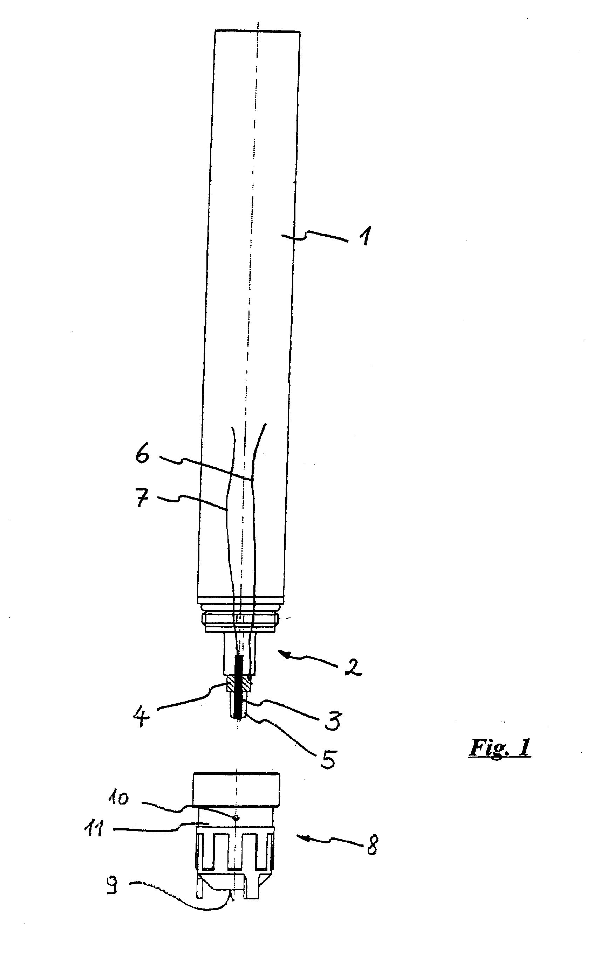

Figur 1 zeigt eine besonders bevorzugte Ausführungsform eines als Zweielektrodensystem ausgebildeten Chloritsensors der vorliegenden Erfindung. Der Sensor hat einen Elektrodenschaft 1, der im wesentlichen zylindrisch ausgebildet ist, und einen Elektrodenkörper 2, der an einem Ende des Elektrodenschafts 1 befestigt, vorzugsweise darin eingeschraubt ist. Der Elektrodenkörper weist eine im wesentlichen stabförmige Arbeitselektrode 3 aus Gold mit einem Durchmesser von etwa 2 mm auf. Im wesentlichen konzentrisch um die Arbeitselektrode 3 aus Gold angeordnet ist eine Gegenelektrode 4, die bei der dargestellten Ausführungsform ein galvanisch mit AgCl belegter Ring bzw. Zylinder aus Silber ist. Zwischen der Arbeitselektrode 3 aus Gold und der Gegenelektrode 4 ist ein Mantel 5 aus einem elektrisch isolierenden Material vorgesehen. Die Kontaktierung der Elektroden 3 und 4 erfolgt über Kontaktdrähte 6 bzw. 7, die von den Elektroden 3 und 4 durch den Elektrodenschaft 1 hindurch zu einer Meßelektronik (nicht dargestellt) geführt sind.

Der in Figur 1 dargestellte Sensor umfaßt weiterhin eine Membrankappe 8, die über den Elektrodenkörper 2 aufgesetzt und vorzugsweise durch Aufschrauben an dem Elektrodenschaft 1 bzw. dem Elektrodenkörper 2 befestigt wird. Die Membrankappe 8 trennt den Innenelektrolyten mit den Arbeits- und Gegenelektroden von der Meßlösung, in welche der Sensor eingetaucht wird. An dem in Figur 1 unten dargestellten Ende ist die Membrankappe 8 mit einer Membran 9 versehen, vorzugsweise einer hydrophilisierten Polyvinylidenfluorid-Membran mit einer Porengröße von 0,5 μm. Bei einer bevorzugten Ausführungsform wird die Membran mittels eines Spannrings in einer Nut auf der Membrankappe 8 eingeklemmt (nicht dargestellt). Der Innenraum der Membrankappe wird mit einem Innenelektrolyten gefüllt, der sowohl mit der Membran 9 als auch den Elektroden 3 und 4 in Kontakt ist. Als Innenelektrolyt eignet sich mit Vorteil eine 50 mM KCl-Lösung, die mit 40 g/L Hydroxyethylcellulose verdickt ist.

An der Membrankappe 8 ist weiterhin eine Druckausgleichsbohrung 10 vorgesehen, die von einem in einer Nut an der Membrankappe 8 umlaufenden Silikonring 11 abgedeckt ist. Der Silikonring 11 verhindert ein Eintreten von Meßlösung durch die Druckausgleichsbohrung 10 in die Membrankappe 8, erlaubt aber einen Austritt von überschüssigem Elektrolyt durch die Druckausgleichsbohrung 10 beim Aufschrauben der Membrankappe 8.

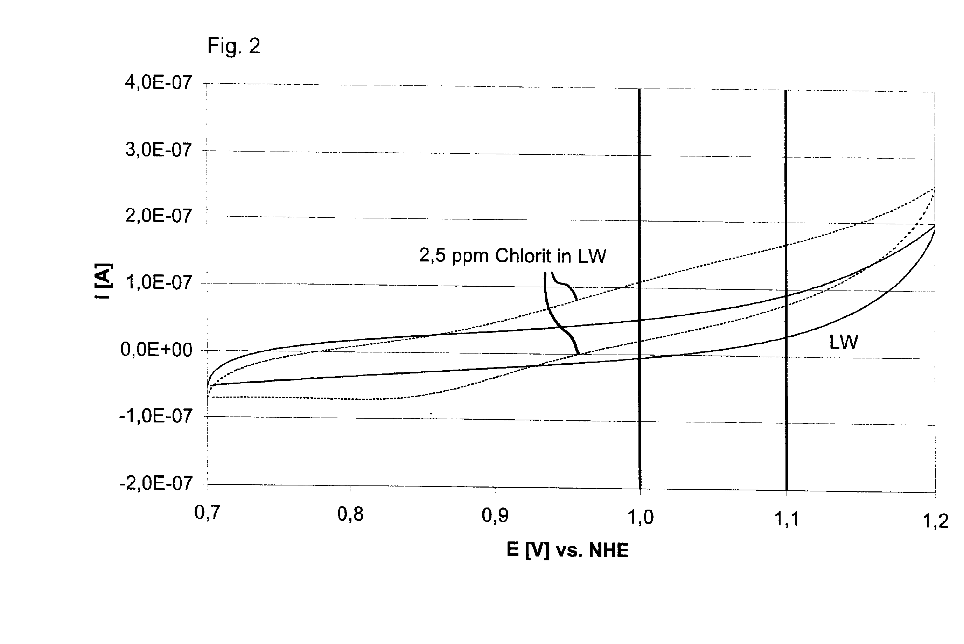

Figur 2 zeigt Cyclovoltammogramme (Strom-Spannungs-Diagramme) in Leitungswasser (LW) ohne Chlorit als sog. Null-Lösung bzw. mit 2,5 ppm Chlorit, jeweils bei einem pH-Wert von 7,2, unter Verwendung des erfindungsgemäßen Chloritsensors gemäß Figur 1.

Aus den Cyclovoltammogrammen kann das Arbeitspotential für den amperometrischen Betrieb des Sensors abgeleitet werden. Der Strom im anodischen Plateaubereich (dargestellt zwischen den vertikalen Linien) zwischen etwa 1000 und 1100 mV gegenüber der Normalwasserstoffelektrode (NHE) ist zu der Konzentration an Chlorit in der Lösung direkt proportional.

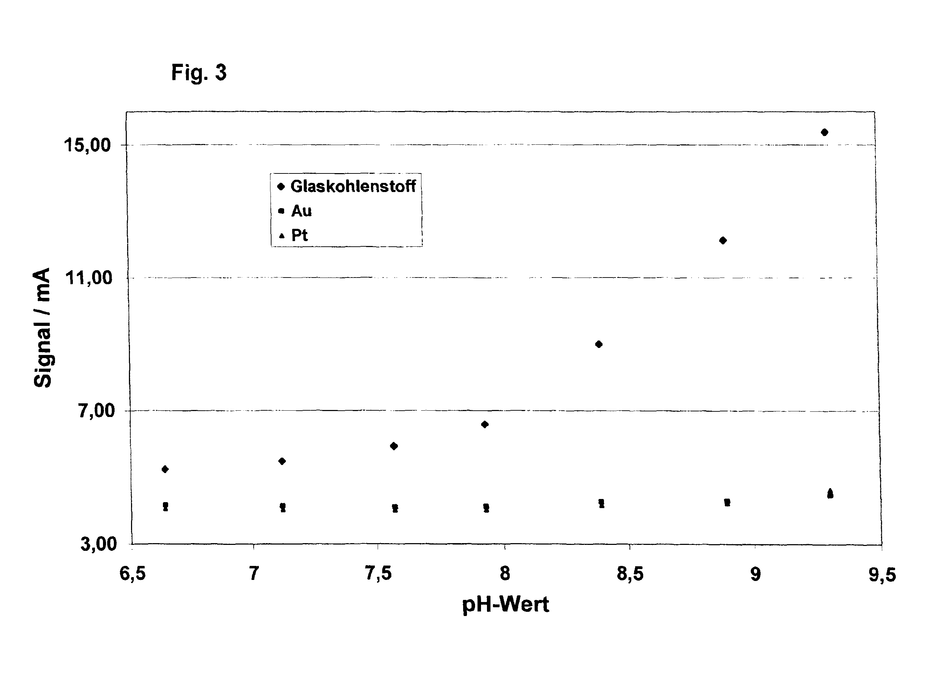

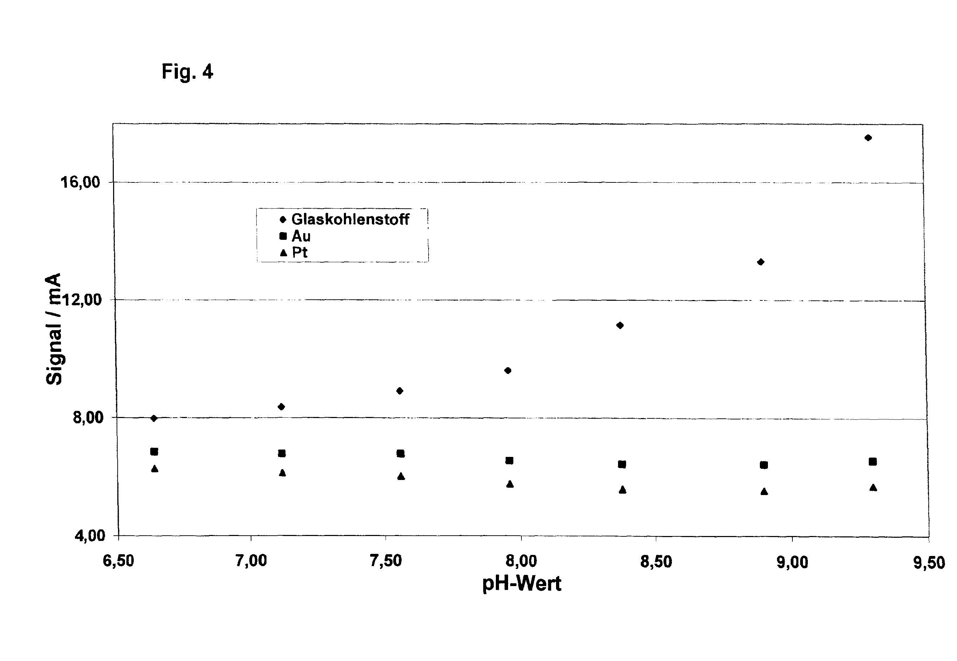

Figur 3 zeigt die Abhängigkeit des Nullpunktsignals und Figur 4 die Abhängigkeit des Chlorit-Signals jeweils vom pH-Wert der Meßlösung (Leitungswasser) unter Verwendung von Sensoren mit drei verschiedenen Arbeitselektrodenmaterialien (Gold, Platin und Glaskohlenstoff), die bei gleicher Versuchsanordnung gemessen wurden. Die Lösung, welche für die in Figur 3 wiedergegebenen Ergebnisse verwendet wurde enthielt kein Chlorit. Die Lösung, welche für die in Figur 4 wiedergegebenen Ergebnisse verwendet wurde enthielt 0,5 ppm Chlorit. Das Arbeitselektrodenpotential betrug jeweils 1000 mV gegenüber NHE. Der jeweilige pH-Wert wurde mit NaOH bzw. HCl eingestellt. Deutlich zeigt die Arbeitselektrode aus Glaskohlenstoff eine starke, nicht lineare Abhängigkeit des Meßsignals vom pH-Wert der Meßlösung. Dem gegenüber sind die gemessenen Potentiale bei den Anordnungen mit Arbeitselektroden aus Platin und Gold über den untersuchten pH-Wertbereich im wesentlichen konstant.

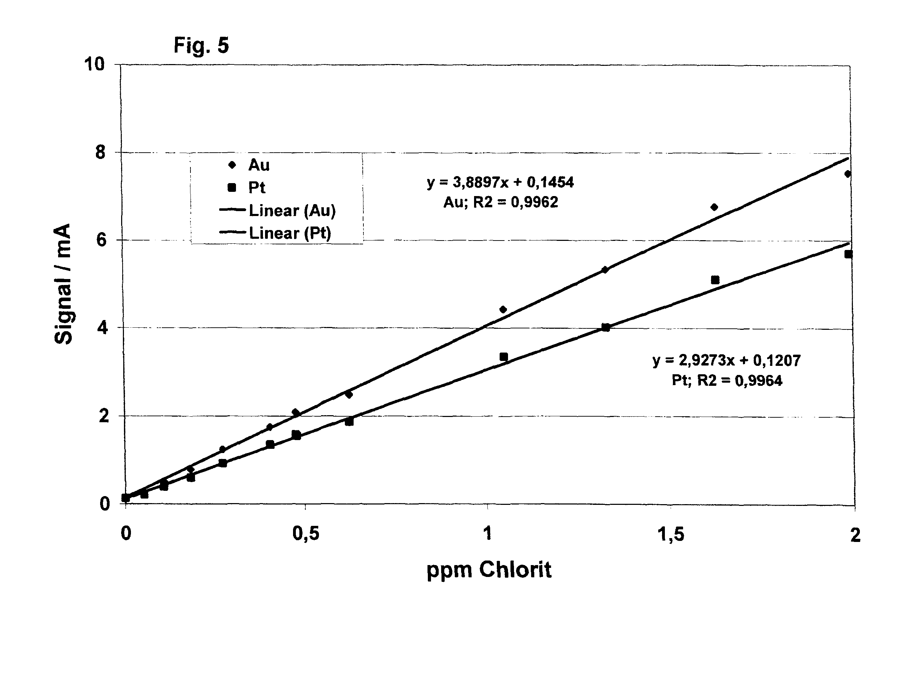

Figur 5 zeigt Kennlinien von Chloritsensoren mit Arbeitselektroden aus Gold bzw. Platin, bei konstantem pH-Wert von 8,0 gemessen wurden, und über einem Chloritbereich von 0 bis 2 ppm Clorit in Leitungswasser. Das Arbeitselektrodenpotential betrug jeweils 1000 mV gegenüber NHE. Deutlich liefert der Sensor mit der Arbeitselektrode aus Gold eine erheblich stärkere Stromzunahme über den Meßbereich von 0 bis 2 ppm Chlorit mit einer steileren Meßkurve gegenüber dem Sensor mit Arbeitselektrode aus Platin. Hieraus werden die Vorteile der Arbeitselektrode aus Gold gegenüber derjenigen aus Platin deutlich, da eine steilere Meßkurve mit größeren Stromunterschieden zwischen verschiedenen Chloritkonzentrationen genauere und empfindlichere Chloritmessungen erlaubt.

Zusammenfassend verdeutlichen die in den Figuren 3 bis 5 gezeigten Ergebnisse die Vorteile der Gold-Arbeitselektrode bei der Chloritbestimmung im niedrigen Konzentrationsbereich gegenüber Arbeitselektroden aus Glaskohlenstoff einerseits und anderen Edelmetallelektroden andererseits. Gegenüber Arbeitselektroden aus Glaskohlenstoff zeichnen sich die Edelmetallelektroden durch eine Unabhängigkeit des Meßsignals vom pH-Wert der Meßlösung aus, zumindest im pH-Wertbereich von etwa 6,0 bis 9,5. Darüber hinaus hat die Arbeitselektrode aus Gold gegenüber derjenigen aus anderem Edelmetall, wie Platin, den Vorteil, daß sie sehr hohe Arbeitspotentiale erlaubt, wodurch die Querempfindlichkeit gegenüber Begleitstoffen ausgeschaltet wird und sie einen erheblich steileren Stromverlauf über die Chloritkonzentration liefert, so daß der Chloritsensor insgesamt genauer und empfindlicher bei niedrigen Chloritkonzentrationen ist. Für die Bestimmung niedriger Mengen des toxischen Chlorits in Leitungswasser ist der erfindungsgemäße Chloritsensor daher hervorragend geeignet und besser als bekannte Vorrichtungen.

**Patentansprüche**

1. Sensor zur voltammetrischen oder amperometrischen Messung der Chloritkonzentration ($ClO_2^-$) in einer wäßrigen Meßlösung, **dadurch gekennzeichnet, daß** der Sensor eine Arbeitselektrode aus Gold aufweist.

2. Sensor nach Anspruch 1, **dadurch gekennzeichnet, daß** die Arbeitselektrode als offene Elektrode für den direkten Kontakt mit der Meßlösung ausgebildet ist.

3. Sensor nach Anspruch 1, **dadurch gekennzeichnet, daß** die Arbeitselektrode von der Meßlösung durch eine Membran räumlich getrennt ist, wobei die Membran vorzugsweise eine hydrophile oder hydrophilisierte Membran ist.

4. Sensor nach Anspruch 3, **dadurch gekennzeichnet, daß** die Membran aus Polyvinylidendifluorid oder Polyethylentherephthalat besteht.

5. Sensor nach Anspruch 3 oder 4, **dadurch gekennzeichnet, daß** die Membran eine Porengröße von 0,1 bis 5 μm, vorzugsweise eine Porengröße von 0,2 bis 1,0 μm, besonders bevorzugt eine Porengröße von etwa 0,5 aufweist.

6. Sensor nach einem der Ansprüche 1 und 3 bis 5, **dadurch gekennzeichnet, daß** die Arbeitselektrode am Sensor von einer Membrankappe umgeben ist, welche die Arbeitselektrode von der Meßlösung trennt, wobei die Membrankappe mit einem Innenelektrolyten gefüllt ist, der mit der Arbeitselektrode in Kontakt ist, und die Membrankappe wenigstens eine Membran aufweist, die den Innenraum der Membrankappe und den Außenraum der Meßlösung trennt.

7. Sensor nach einem der Ansprüche 1 bis 6, **dadurch gekennzeichnet, daß** er wenigstens eine weitere Elektrode als Gegenelektrode, vorzugsweise eine mit Silberchlorid belegte Silberelektrode aufweist.

8. Sensor nach einem der Ansprüche 1 bis 7, **dadurch gekennzeichnet, daß** er eine mit Silberchlorid belegte Silberelektrode als nicht stromdurchflossene Referenzelektrode zum Anlegen eines Arbeitspotentials und eine weitere Elektrode als stromdurchflossene Gegenelektrode aufweist.

9. Verfahren zur Messung der Chloritkonzentration in einer wäßrigen Meßlösung, bei dem man den Sensor nach einem der Ansprüche 1 bis 8 verwendet und zwischen der Arbeitselektrode und der Gegenelektrode als Arbeitsspannung ein konstantes anodisches Potential von +900 bis +1150 mV gegenüber der Normalwasserstoffelektrode anlegt und den bei der Arbeitsspannung fließenden Strom mißt.

10. Verfahren nach Anspruch 9, **dadurch gekennzeichnet, daß** die Arbeitsspannung von +1000 bis +1100 mV gegenüber der Normalwasserstoffelektrode beträgt.

11. Verwendung von Gold als Arbeitselektrode in einem Sensor zur amperometrischen Messung der Chloritkonzentration ($ClO_2^-$) in einer wäßrigen Lösung.

*Fig. 1*

Fig. 2

Fig. 3

Fig. 4

Fig. 5

**Europäisches Patentamt**

# EUROPÄISCHER RECHERCHENBERICHT

Nummer der Anmeldung

EP 04 10 1710

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | PATENT ABSTRACTS OF JAPAN vol. 015, no. 079 (P-1170), 25. Februar 1991 (1991-02-25) -& JP 02 296145 A (TOA DENPA KOGYO KK), 6. Dezember 1990 (1990-12-06) Fig. 2 & 3 des Patents: Gold als Elektrodenmaterial, Potential etwa 1V * Zusammenfassung * | 1,2,7-11 | G01N27/49 G01N27/403 |
| Y | | 3-6 | |
| Y | EP 0 740 149 A (PROMINENT DOSIERTECHNIK GMBH) 30. Oktober 1996 (1996-10-30) * Zusammenfassung; Abbildung 1 * * Spalte 5, Zeile 19 - Zeile 46 * | 3-6 | |
| X | PATENT ABSTRACTS OF JAPAN vol. 015, no. 079 (P-1170), 25. Februar 1991 (1991-02-25) -& JP 02 296146 A (TOA DENPA KOGYO KK), 6. Dezember 1990 (1990-12-06) Fig. 2 & 4 des Patents: Gold als Elektrodenmaterial, Potential etwa 1V * Zusammenfassung * | 1,2,7-11 | |
| Y | | 3-6 | **RECHERCHIERTE SACHGEBIETE (Int.Cl.7)** G01N |
| Y | DE 44 39 285 A (REIS GERHARD DR) 15. Mai 1996 (1996-05-15) * Zusammenfassung; Abbildung 1 * * Spalte 1, Zeile 16 - Zeile 43 * | 3-6 | |

-/--

Der vorliegende Recherchenbericht wurde für alle Patentansprüche erstellt

| Recherchenort | Abschlußdatum der Recherche | Prüfer |
|---|---|---|
| MÜNCHEN | 23. September 2004 | Hanisch, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**Europäisches Patentamt**

# EUROPÄISCHER RECHERCHENBERICHT

Nummer der Anmeldung

EP 04 10 1710

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | DATABASE EMBASE [Online] ELSEVIER SCIENCE PUBLISHERS, AMSTERDAM, NL; 1989 DENIS M ET AL: "Continuous determination of residual chlorite in water" Database accession no. EMB-1989269697 XP002297529 * Zusammenfassung * -& ANALYTICA CHIMICA ACTA, NETHERLANDS, Bd. 226, Nr. 1, 1989, Seiten 121-128, XP009036990 ISSN: 0003-2670 --- | 1,11 | |
| X | EP 0 563 690 A (PROMINENT DOSIERTECHNIK GMBH) 6. Oktober 1993 (1993-10-06) * Zusammenfassung; Abbildungen 1,2 * * Spalte 6, Zeile 35 - Spalte 7, Zeile 9 * * Spalte 8, Zeile 16 - Zeile 19 * * Spalte 8, Zeile 36 - Zeile 38 * | 1,3,6-8 | |
| A | ----- | 3-6 | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| MÜNCHEN | 23. September 2004 | Hanisch, C |

**EP 1 484 606 A1**

## ANHANG ZUM EUROPÄISCHEN RECHERCHENBERICHT
## ÜBER DIE EUROPÄISCHE PATENTANMELDUNG NR.

EP 04 10 1710

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-09-2004

| Im Recherchenbericht angeführtes Patentdokument | | Datum der Veröffentlichung | Mitglied(er) der Patentfamilie | | Datum der Veröffentlichung |
|---|---|---|---|---|---|
| JP 02296145 | A | 06-12-1990 | JP | 2023568 C | 26-02-1996 |
| | | | JP | 7046093 B | 17-05-1995 |
| EP 0740149 | A | 30-10-1996 | DE | 19515392 A1 | 07-11-1996 |
| | | | DE | 59610015 D1 | 06-02-2003 |
| | | | EP | 0740149 A1 | 30-10-1996 |
| | | | ES | 2186743 T3 | 16-05-2003 |
| | | | JP | 2872633 B2 | 17-03-1999 |
| | | | JP | 8304331 A | 22-11-1996 |
| | | | US | 5725747 A | 10-03-1998 |
| JP 02296146 | A | 06-12-1990 | JP | 2008061 C | 11-01-1996 |
| | | | JP | 7031157 B | 10-04-1995 |
| DE 4439285 | A | 15-05-1996 | DE | 4439285 A1 | 15-05-1996 |
| EP 0563690 | A | 06-10-1993 | DE | 4211198 A1 | 07-10-1993 |
| | | | EP | 0563690 A1 | 06-10-1993 |

Für nähere Einzelheiten zu diesem Anhang : siehe Amtsblatt des Europäischen Patentamts, Nr.12/82

14